# FASCICULE DE BREVET EUROPEEN

(11) **EP 2 555 698 B1**
(45) Date de publication et mention de la délivrance du brevet: **02.04.2014**
(21) Numéro de dépôt: 11725147.0
(22) Date de dépôt: 10.05.2011
(51) Int. Cl.: A61B 17/70

(54) **DISPOSITIF DE MAINTIEN A ELEMENT LONGILIGNE ADAPTE POUR MAINTENIR UN ECARTEMENT INTERVERTEBRAL DETERMINE**
AUFRECHTERHALTUNGSVORRICHTUNG MIT EINER SCHMALEN KOMPONENTE ZUR AUFRECHTERHALTUNG EINES VORDEFINIERTEN INTERVERTEBRALEN ABSTANDS
MAINTAINING DEVICE COMPRISING A SLENDER COMPONENT SUITABLE FOR MAINTAINING A PREDETERMINED INTERVERTEBRAL SPACING

(30) Priorité: 23.06.2010 FR 1055017
(43) Date de publication de la demande: 13.02.2013
(73) Titulaire: Cousin Biotech, 59117 Wervicq Sud (FR)
(72) Inventeur: GUIZZARDI, Giancarlo, I-50141 Firenze (IT); BARDEL, Frédéric, F-59116 Houplines (FR); DENEUVILLERS, Guy, F-62155 Merlimont (FR)
(74) Mandataire: Balesta, Pierre
(86) Numéro de dépôt international: PCT/FR2011/051048
(87) Numéro de publication internationale: WO 2011/161341

(56) Documents cités:
- EP-B1- 1 343 424
- WO-A1-2009/149407
- CA-A1- 2 106 808
- FR-A1- 2 704 745
- FR-A1- 2 921 248
- US-A- 5 683 404

## Description

La présente invention concerne le domaine technique des dispositifs de maintien qui sont adaptés pour maintenir un écartement intervertébral prédéterminé.

Ce genre de dispositifs de maintien met en oeuvre un ligament ou un élément longiligne apte à maintenir un écartement intervertébral déterminé entre deux vertèbres adjacentes lors des mouvements d'extension, de flexion, mais également dans la position de la lordose naturelle.

Ce type d'élément longiligne peut être utilisé seul ou en combinaison avec un dispositif de soutien intervertébral tel qu'une cale intervertébrale.

Un tel dispositif de soutien intervertébral, disposé entre les apophyses épineuses et/ou les lames, selon notamment l'état des ligaments naturels postérieurs, peut apporter un effet amortissant entre deux vertèbres adjacentes.

De tels éléments longilignes présentent, une fois leurs deux extrémités solidarisées pour former une boucle apte à entourer les apophyses épineuses et/ou les lames de deux vertèbres adjacentes, une résistance à rupture [daN] très inférieure à la résistance à rupture [daN] obtenue classiquement par traction sur les deux extrémités libres du ligament.

En effet, le mode de fixation sélectionné pour solidariser les deux extrémités de l'élément longiligne forme une zone de faiblesse présentant une résistance à rupture moindre que celle de l'élément longiligne.

Les différents systèmes d'attache des deux extrémités libres d'un élément longiligne sont les suivants :
- la réalisation d'un noeud simple ou double des deux extrémités de l'élément longiligne ;
- le positionnement d'une boucle à l'une des extrémités de l'élément longiligne en sorte de passer l'autre extrémité libre dans la boucle, puis d'effectuer une couture de l'extrémité libre passée à travers la boucle sur une portion de l'élément longiligne ;
- le passage de l'élément longiligne dans une cale intervertébrale de forme complexe comportant de nombreuses pièces individuelles telles que la cale décrite dans le document EP 1.343.424 ;
- le passage d'une ou des deux extrémités libres de l'élément longiligne dans une bague métallique déformable, puis la compression de la bague en sorte de serrer les deux extrémités ensemble.

Ces systèmes d'attache des extrémités libres des éléments longilignes ont pour inconvénients qu'ils sont complexes à mettre en oeuvre lors d'une intervention chirurgicale.

FR2704745 comprend une plaque de matériau résistant et malléable, apte à être fixée à l'une des extrémités du ligament, et des moyens, solidaires de la plaque, pour l'engagement et la rétention de l'autre extrémité du ligament, ladite plaque étant apte à être engagée, avec le ligament, derrière l'apophyse épineuse de l'une des deux vertèbres délimitant l'articulation à traiter et à être repliée de part et d'autre de celle-ci pour former un crochet de maintien en position desdits moyens d'engagement et de rétention, et ces derniers étant aptes à recevoir l'autre extrémité du ligament après engagement autour de l'autre apophyse épineuse, en permettant le coulissement de cette extrémité dans son sens d'engagement et en empêchant ce coulissement dans le sens inverse.

Par ailleurs, ils sont non-réversibles pour la plupart, et présentent une faible résistance à rupture par rapport à celle de l'élément longiligne.

De plus, lorsque le système d'attache est complexe et fait intervenir de nombreuses pièces individuelles, il existe un risque au regard des mouvements de flexion et d'extension auxquels sont soumis les deux vertèbres adjacentes.

En effet, il est possible que l'une des pièces se détache, ce qui serait rédhibitoire en termes d'utilisation. En effet, la zone d'implantation qui est au plus près de la moelle épinière est très sensible.

Ces systèmes présentent également pour inconvénient que, durant le temps de solidarisation des deux extrémités de l'élément longiligne délimitant une boucle d'un périmètre donné disposée autour de deux apophyses épineuses et/ou deux lames adjacentes, la tension exercée sur les extrémités peut varier et donc modifier le périmètre de la boucle formée, engendrant alors des imprécisions ; ceci est d'autant plus vrai que la solidarisation est réalisée de façon manuelle.

La présente invention a pour but de pallier aux différents inconvénients précités.

A cet effet, l'objet de la présente invention concerne un dispositif de maintien adapté pour maintenir un écart intervertébral déterminé palliant tout ou partie des problèmes précités.

Plus précisément, le dispositif de maintien selon la présente invention comporte un élément longiligne rond ou sensiblement plat de largeur principale l, ayant des première et seconde extrémités, apte à être disposé autour des apophyses épineuses.

Le dispositif de maintien selon la présente invention comporte en outre un support rigide comprenant deux parties latérales montées entre des parties avant et arrière.

La première extrémité est solidarisée ou apte à être solidarisée à la partie arrière et la seconde extrémité reste libre.

Le dispositif de maintien selon la présente invention comporte également un élément de blocage monté coulissant sur les parties latérales et délimitant des zones de passage avant et arrière respectivement avec les parties avant et arrière.

Cet agencement permet le passage de la seconde extrémité libre dans la zone de passage arrière puis dans la zone de passage avant en enveloppant partiellement l'élément de blocage en sorte que :
a) la portion de l'élément longiligne s'étendant sensiblement entre sa première extrémité et l'élément de blocage forme une boucle de périmètre donné p ; et que
b) l'application de tensions opposées sur les parois internes de la boucle provoque le déplacement de l'élément de blocage vers la partie avant et le blocage de la portion d'élément longiligne dans la zone de passage avant entre l'élément de blocage et la partie avant du support.

Avantageusement, le support et l'élément de blocage assurent une solidarisation mécanique solide des deux extrémités de l'élément longiligne puisque la résistance à rupture [daN] obtenue est au moins de l'ordre d'environ 50%, voire plus, de la résistance à rupture [daN] de l'élément longiligne.

De plus, lorsque le chirurgien enfile la seconde extrémité libre dans la zone de passage arrière puis dans la zone de passage avant, afin de former une boucle de périmètre donné p, entourant par exemple deux apophyses épineuses adjacentes, les forces opposées exercées par les apophyses épineuses lors des mouvements de flexion du rachis contre les parois internes de la boucle formée ne peuvent allonger la boucle et modifier le périmètre qui lui a été attribué préalablement par le chirurgien.

Ceci permet donc de maintenir un écart intervertébral déterminé entre les deux apophyses épineuses traitées. Il n'y a ainsi pas d'imprécision lors de la solidarisation des deux extrémités de l'élément longiligne pour la formation d'une boucle.

Avantageusement, la solidarisation des première et seconde extrémités est totalement réversible en exerçant une traction sur la portion d'élément longiligne en périphérie de la zone de passage arrière dans une direction opposée à la zone de passage avant tout en maintenant le support.

De plus, il est possible d'exercer une traction sur la portion d'élément longiligne en périphérie de la zone de passage arrière dans une direction opposée à ladite zone de passage arrière afin de diminuer le périmètre de la boucle formée.

Le dispositif de maintien selon la présente invention permet ainsi d'offrir une solidarisation réversible des première et seconde extrémités de l'élément longiligne ainsi qu'un ajustement aisé du périmètre de la boucle formée.

Avantageusement, l'élément longiligne est sensiblement plat en sorte que les frottements générés par le passage dudit élément sur les parties avant, arrière et l'élément de blocage participent au blocage de l'élément longiligne dans les zones de passage avant et arrière.

Dans une variante, la distance interne d séparant les parties latérales est sensiblement de l'ordre de celle de la largeur principale l dudit élément longiligne ou inférieure.

Cette disposition permet d'augmenter les zones de frottements entre l'élément longiligne et les parties avant, arrière, latérales et l'élément de blocage afin d'améliorer le blocage de l'élément longiligne dans les zones de passage avant et arrière.

Dans une variante, la partie avant du support est dimensionnée et agencée par rapport à l'élément de blocage en sorte de faire office de butée avant pour ledit élément de blocage. De préférence, les bords gauche et droit de la partie avant se projettent de part et d'autre des parties latérales et font office de butée aux bords gauche et droit de l'élément de blocage.

Cet agencement des éléments permet de limiter le serrage de l'élément longiligne, et de sécuriser le système en limitant le cisaillement de l'élément longiligne.

Dans une variante, l'élément de blocage comprend une ouverture centrale dont les bords latéraux internes sont ménagés en sorte de recevoir les parties latérales du support et faire office de guide pour la coulisse de l'élément de blocage sur les parties latérales entre les parties avant et arrière du support.

Dans une variante, la périphérie supérieure de l'ouverture centrale débouchant vers la partie avant est délimitée par des bords gauche et droit, et le bord gauche se projette de la périphérie supérieure, en sorte que seul le bord gauche de l'élément de blocage vient en butée contre la partie avant du support ménageant un jeu entre le bord droit et la partie avant.

Le jeu ménagé entre le bord droit et la partie avant du support permet de diminuer la force du pincement.

Ceci permet donc de limiter le cisaillement de l'élément longiligne pincé entre l'élément de blocage et la partie avant du support lorsque des tensions opposées sont exercées sur les parois internes de la boucle.

La résistance à rupture de l'élément longiligne dont les extrémités sont solidarisées aux moyens du support et de l'élément de blocage autorise donc une plus grande résistance à la rupture.

Dans une variante, l'élément de blocage comprend des rainures verticales, orientées de la partie arrière vers la partie avant, et débouchant sur la périphérie supérieure, et éventuellement sur la périphérie inférieure, de l'ouverture centrale en sorte de ménager des dents de pincement sur la périphérie supérieure, et éventuellement sur la périphérie inférieure.

Le blocage de l'élément longiligne dans les zones de passage avant et arrière est ainsi amélioré.

Dans une variante, l'ouverture centrale débouchant vers la partie avant du support comporte, selon au moins l'un de ses bords gauche ou droit, des dents en forme de dents de scie.

Bien évidemment, on comprendra que la surface du support peut également être lisse ou rugueuse pour permettre le blocage.

Dans une variante, les zones angulaires internes du support délimitées entre les parties avant et/ou arrière et l'une ou l'autre des deux parties latérales sont arrondies en sorte de diminuer le cisaillement exercé sur les portions d'élément longiligne en contact à friction avec les zones angulaires.

Dans une variante, le support est dans un matériau sélectionné seul ou en combinaison parmi les matériaux suivants : acier inoxydable, titane, céramique, polypropylène, polyéthylène, polyéthylène haute densité, fibre de carbone, polyétherétherkétone (PEEK), chrome cobalt, etc.

Dans une variante, l'élément longiligne est obtenu par la mise en forme de fils multi-filamentaires et/ou de mono-filaments par tissage, tricotage ou tressage, de préférence par tressage de fils multi-filamentaires.

Cette mise en forme permet la formation de surfaces planes en relief augmentant les forces de frottement générées entre l'élément longiligne et le support et l'élément de blocage, et corrélativement améliorant le blocage de l'élément longiligne dans les zones de passage avant et arrière.

De plus, les fils multi-filamentaires permettent d'augmenter la résistance à rupture de l'élément longiligne comparativement à des mono-filaments ou des éléments longilignes pleins.

Dans une variante, l'élément longiligne est dans un matériau sélectionné seul ou en combinaison parmi les matériaux suivants : polypropylène, polyéthylène téréphtalate, polyéthylène, polyéthylène haute densité, polyamide 6-6, 4-6 ou 12, polyuréthane, polyétherétherkétone (PEEK).

Dans une variante, l'élément longiligne est une tresse tubulaire dont les première et seconde extrémités ont été cousues pour la mise en forme à plat de la tresse tubulaire.

L'élément longiligne présente ainsi une résistance à rupture améliorée puisque les tensions s'exercent à la fois sur deux épaisseurs textiles interconnectées.

Dans une variante, la seconde extrémité est confectionnée en forme de pointe ; de préférence elle comporte une boucle de préhension.

Dans une variante, l'élément longiligne comprend une zone centrale disposée entre des zones latérales et les zones latérales présentent un coefficient de friction moins élevé que la zone centrale afin d'améliorer la résistance à rupture de l'élément longiligne dont les extrémités sont solidarisées sur le support et l'élément de blocage tout en préservant les frottements générés pour améliorer le blocage de l'élément longiligne.

De préférence, la zone centrale est mise en forme à partir de fils multi-filamentaires et/ou de mono-filaments, de préférence par tressage, à base de polyéthylène téréphtalate, de polypropylène ou de polyamide tandis que les zones latérales sont mises en forme à partir de fils multi-filamentaires et/ou de mono-filaments, de préférence par tressage, à base de polyéthylène haute densité, de polytétrafluoroéthylène.

Lesdites zones latérales peuvent être enduites d'un matériau ayant un faible coefficient de frottement, tel que de la silicone ou du PTFE.

Dans une variante, le dispositif comprend un dispositif de soutien intervertébral tel qu'une cale intervertébrale dont au moins la partie antérieure est apte à être disposée entre les lames sus et sous-jacentes respectivement de deux vertèbres sus et sous-jacentes.

Dans une variante, le dispositif de soutien intervertébral comporte au moins un moyen d'attache pour l'ancrage de l'élément longiligne.

De préférence, ce moyen d'attache est disposé sur l'une de ses parois latérales.

La présente invention sera mieux comprise à la lecture d'un exemple de réalisation, cité à titre non limitatif, et dans lesquels:
- la figure 1A est une représentation schématique d'un premier exemple de support et d'élément de blocage selon l'invention ;
- la figure 1B est une représentation schématique d'un second exemple de support et d'élément de blocage selon l'invention ;
- la figure 1C est une représentation schématique d'un troisième exemple de support et d'élément de blocage selon l'invention ;
- la figure 2 est une représentation schématique en perspective d'un dispositif de maintien d'un écart intervertébral maximum en fonctionnement comportant le support et l'élément de blocage représentés à la figure 1C ;
- les figures 3-5 sont des représentations schématiques du dispositif de maintien intervertébral représenté à la figure 2 dans différentes position du rachis : en lordose naturelle (figure 3), en flexion (figure 4) et en extension (figure 5).

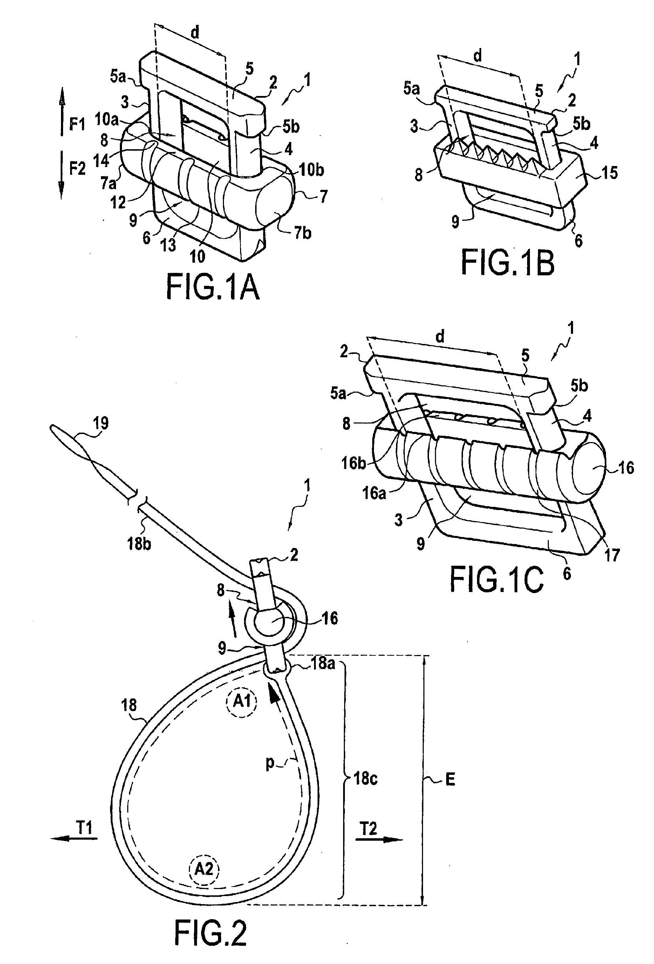

Le dispositif de maintien 1 représenté partiellement à la figure 1A comprend un support rigide 2, qui se présente dans cet exemple précis sous forme de cadre.

Dans l'exemple décrit ici, le support 2 selon la présente invention comprend deux parties latérales 3 et 4 montées entre des parties avant 5 et arrière 6 ainsi qu'un élément de blocage 7 monté coulissant sur les parties latérales 3,4 et délimitant des zones de passage avant 8 et arrière 9 respectivement avec les parties avant 5 et arrière 6.

Les parties latérales 3 et 4 sont séparées d'une distance interne d.

Les zones angulaires internes du support 2 délimitées entre les parties avant 5 et/ou arrière 6 et l'une ou l'autre des deux parties latérales 3 et 4 sont arrondies.

Dans l'exemple décrit ici, la partie avant 5 du support 2 est dimensionnée et agencée par rapport à l'élément de blocage 7 en sorte de faire office de butée avant pour l'élément de blocage 7.

Dans cet exemple précis, les bords gauche 5a et droit 5b de la partie avant 5 se projetant de part et d'autre des parties latérales 3 et 4 font office de butée aux bords gauche 7a et droit 7b de l'élément de blocage 7.

L'élément de blocage 7 comprend en outre une ouverture centrale 10 dont les bords latéraux internes 10a et 10b sont ménagés en sorte de a) recevoir les parties latérales 3 et 4 du support 2 et b) faire office de guide pour la coulisse de l'élément de blocage selon les directions F1 et F2 entre les parties avant 5 et arrière 6.

L'élément de blocage 7 comprend des rainures verticales 11, orientées de la partie arrière 6 vers la partie avant 5, débouchant sur les périphéries supérieure 12 et inférieure 13 de l'ouverture centrale 10 en sorte de ménager des dents de pincement 14.

Aux figures 1B et 1C, seuls les éléments de blocage 15 et 16 diffèrent de l'élément de blocage 7 représenté à la figure 1A.

L'élément de blocage 15 comporte au niveau de l'ouverture centrale 10 débouchant vers la partie avant 5 du support 2 selon le bord gauche 15a des dents en forme de scie.

A la figure 1C, l'ouverture centrale 10 de l'élément de blocage 16 débouchant vers la partie avant 5 est délimitée par des bords gauche 16a et droit 16b.

Plus précisément, dans cet exemple, le bord gauche 16a se projette de l'ouverture 10 en sorte que seul ce dernier vienne en contact avec la partie avant 5 du support 2 lorsque l'élément de blocage 16 coulisse vers la partie avant 5, ménageant de ce fait un jeu entre le bord droit 16b et la partie avant 5.

L'élément de blocage 16 comprend des rainures verticales 17 similaires aux rainures verticales 11 représentées à la figure 1A.

Le dispositif de maintien 1 représenté à la figure 2 comprend un support 2 et un élément de blocage 16 tels que représentés en figure 1C.

Le dispositif 1 comprend également un élément longiligne 18 sensiblement plat ayant une première extrémité 18a solidarisée à la partie arrière 6 du support 2 et une seconde extrémité 18b libre comportant une boucle de préhension 19.

Dans cet exemple précis, l'élément longiligne 18 est une tresse tubulaire obtenue à partir des fils multi-filamentaires et dont les extrémités 18a et 18b ont été cousues en sorte d'aplatir la tresse.

Dans cet exemple précis, la largeur l de l'élément longiligne 18 est sensiblement de l'ordre de la distance interne d séparant les parties latérales 3 et 4 en sorte de ménager des frottements avec les différentes parties 3, 4, 5 et 6 du support 2 lors du passage de l'élément longiligne 18.

Cet agencement améliore ainsi le blocage de l'élément longiligne 18.

En fonctionnement, la seconde extrémité libre 18b passe dans la zone de passage arrière 9 puis la zone de passage avant 8.

Lors de ce passage, la seconde extrémité libre 18b enveloppe partiellement l'élément de blocage 16 en sorte que la portion 18c de l'élément longiligne 18 s'étendant sensiblement entre sa première extrémité 18a et l'élément de blocage 16 forme une boucle de périmètre donné p et de hauteur maximale E enveloppant, dans cet exemple précis, les apophyses épineuses sus A1 et sous A2 jacentes.

L'application de tensions opposées T1 et T2 lors des mouvements de flexion ou d'extension du rachis sur les parois internes de la portion de boucle 18c provoque le déplacement dudit élément de blocage 16 vers la partie avant 5 du support 2 et le blocage de l'élément longiligne 18 dans la zone de passage avant 8 entre l'élément de blocage 16 et la partie avant 5 du support 2.

Les rainures verticales 17 de l'élément de blocage 16 forment des dents de pincement améliorant le serrage et donc le blocage de l'élément longiligne 18 dans la zone de passage avant 8.

Le bord gauche 16a surélevé permet de ménager un jeu avec la partie avant 5 dans la zone de passage avant 8 diminuant ainsi le cisaillement exercé sur l'élément longiligne 18.

Lors d'une intervention, le chirurgien dispose ainsi aisément le dispositif de maintien 1 entre deux parties du rachis sans avoir à exercer une tension pendant la solidarisation de la boucle 18c.

Cette solidarisation est de plus réversible, et le périmètre interne p de la boucle 18c et donc l'écart maximum E sont également facilement réglables.

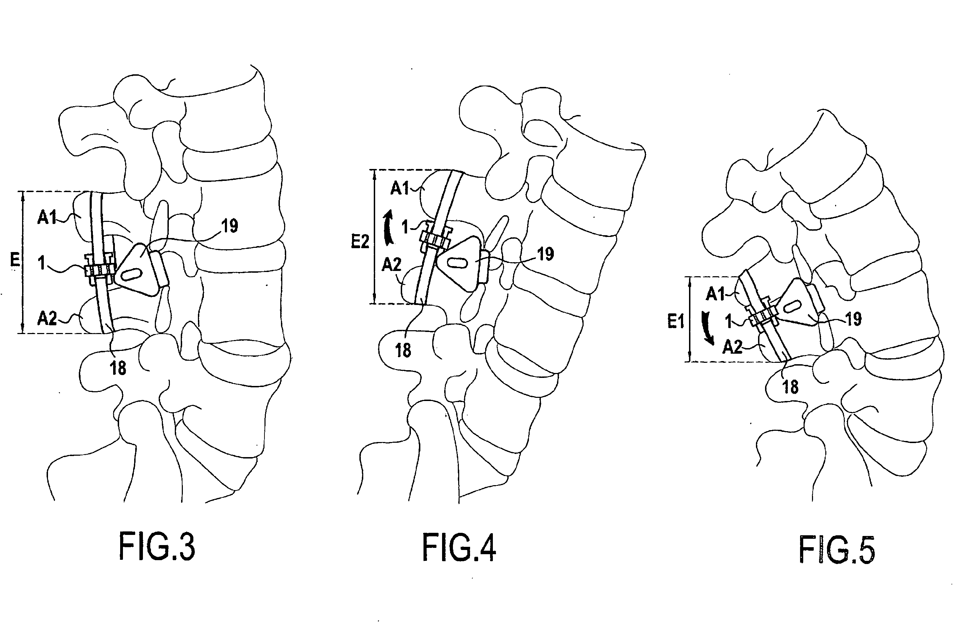

La figure 3 représente le dispositif de maintien 1 dans la position de lordose naturelle avec un dispositif de soutien intervertébral 19, dans cet exemple précis une cale 19. La distance maintenue E entre les apophyses épineuses A1, A2 adjacentes est sensiblement égale à l'écart maximum E assuré par le dispositif de maintien 1.

A la figure 5, le rachis étant dans une position d'extension, les apophyses épineuses A1 et A2 se rapprochent l'une de l'autre et la distance E1 est inférieure à E.

A la figure 4, lorsque le rachis est en flexion, les apophyses épineuses adjacentes A1 et A2 s'éloignent l'une de l'autre mettant en tension le dispositif de maintien 1 qui maintient un écart intervertébral maximum E2 déterminé par le chirurgien lors de la pose du dispositif 1.

Plus précisément, le dispositif de maintien 1 dans cette configuration présente une déformation élastique correspondant jusqu'à un pourcentage d'allongement élastique de l'élément longiligne.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

## Revendications

1. Dispositif de maintien (1) adapté pour maintenir un écartement intervertébral déterminé comportant un élément longiligne (18), de préférence sensiblement plat, de largeur principale déterminée (l), ayant une première (18a) et une seconde (18b) extrémités, apte à être disposé autour des apophyses épineuses, ledit dispositif de maintien (1) étant **caractérisé en ce qu'**il comporte en outre :
a) un support rigide (2) comprenant deux parties latérales (3, 4) montées entre des parties avant (5) et arrière (6), ladite première extrémité (18a) étant solidarisée ou apte à être solidarisée à ladite partie arrière (6) et ladite seconde extrémité (18b) restant libre,
b) un élément de blocage (7) monté coulissant sur lesdites parties latérales (3, 4) et délimitant des zones de passage avant (8) et arrière (9) respectivement avec les parties avant (5) et arrière (6) permettant le passage de ladite seconde extrémité libre (18b) dans la zone de passage arrière (9) puis la zone de passage avant (8) en enveloppant partiellement ledit élément de blocage (7) en sorte que la portion (18c) dudit élément longiligne (18) s'étendant sensiblement entre sa première extrémité (18a) et l'élément de blocage (7) forme une boucle de périmètre donné (p) et en que l'application de tensions opposées (T1, T2) sur les parois internes de la boucle provoque le déplacement dudit élément de blocage (7) vers ladite partie avant (5) et le blocage de la portion (18c) de l'élément longiligne (18) dans la zone de passage avant (8) entre ledit élément de blocage (7) et la partie avant (5) du support (2).

2. Dispositif de maintien (1) selon la revendication 1, **caractérisé en ce que** la distance interne (d) séparant les parties latérales (3, 4) est sensiblement de l'ordre de celle de la largeur principale (l) dudit élément longiligne (18) ou inférieure.

3. Dispositif de maintien (1) selon l'une ou l'autre des revendications 1 et 2, **caractérisé en ce que** la partie avant (5) du support (2) est dimensionnée et agencée par rapport à l'élément de blocage (7) en sorte de faire office de butée avant pour ledit élément de blocage (7), de préférence les bords gauche (5a) et droit (5b) de la partie avant (5) se projetant de part et d'autre des parties latérales (3, 4) font office de butée aux bords gauche et droit de l'élément de blocage (7).

4. Dispositif de maintien (1) selon l'une des revendications précédentes, **caractérisé en ce que** l'élément de blocage (7) comprend une ouverture centrale (10) dont les bords latéraux internes (10a, 10b) sont ménagés en sorte de recevoir les parties latérales (3, 4) du support (2) et faire office de guide pour la coulisse de l'élément de blocage (7) sur les parties latérales (3, 4) entre les parties avant (5) et arrière (6) du support.

5. Dispositif de maintien (1) selon la revendication 4, **caractérisé en ce que** la périphérie supérieure (12) de l'ouverture centrale (10) débouchant vers la partie avant (5) est délimitée par des bords gauche (16a) et droit (16b) et **en ce que** le bord gauche (16a) se projette de ladite périphérie supérieure (12) en sorte que seul le bord gauche (16a) de l'élément de blocage (16) vienne en butée contre la partie avant (5) du support (2) ménageant un jeu entre le bord droit (16b) et la partie avant (5).

6. Dispositif de maintien (1) selon l'une ou l'autre des revendications 4 et 5, **caractérisé en ce que** l'élément de blocage comprend des rainures verticales (17), orientées de la partie arrière (6) vers la partie avant (5), débouchant sur la périphérie supérieure (12), et éventuellement la périphérie inférieure (13), de l'ouverture centrale (10) en sorte de ménager des dents de pincement (14) sur la périphérie supérieure (12), et éventuellement la périphérie inférieure (13).

7. Dispositif de maintien (1) selon l'une des revendications 1 à 4, **caractérisé en ce que** l'ouverture centrale (10) débouchant vers la partie avant (5) du support (2) comporte selon au moins l'un de ses bords gauche (16a) ou droit (16b) des dents en forme de dents de scie.

8. Dispositif de maintien (1) selon l'une des revendications précédentes, **caractérisé en ce que** les zones angulaires internes du support délimitées entre les parties avant et/ou arrière et l'une ou l'autre des deux parties latérales sont arrondies.

9. Dispositif de maintien (1) selon l'une des revendications précédentes, **caractérisé en ce que** le support est dans un matériau sélectionné seul ou en combinaison parmi les matériaux suivants : acier inoxydable, titane, céramique, polypropylène, polyéthylène, polyéthylène haute densité, polyétherétherkétone PEEK, chrome cobalt, PET.

10. Dispositif de maintien (1) selon l'une des revendications précédentes, **caractérisé en ce que** l'élément longiligne (18) est obtenu par la mise en forme de fils multi-filamentaires et/ou de monofilaments par tissage, tricotage ou tressage, de préférence par tressage de fils multi-filamentaires.

11. Dispositif de maintien (1) selon l'une des revendications précédentes, **caractérisé en ce que** l'élément longiligne (18) est dans un matériau sélectionné seul ou en combinaison parmi les matériaux suivants : polypropylène, polyéthylène téréphtalate, polyéthylène, polyéthylène haute densité, polyamide 6-6, 4-6 ou 12, polyuréthane, polyétherétherkétone (PEEK),

12. Dispositif de maintien (1) selon l'une des revendications précédentes, **caractérisé en ce que** l'élément longiligne (18) est une tresse tubulaire dont les première (18a) et seconde (18b) extrémités ont été cousues pour la mise en forme à plat de la tresse tubulaire.

13. Dispositif de maintien (1) selon l'une des revendications précédentes, **caractérisé en ce que** la seconde extrémité (18b) est confectionnée en forme de pointe, de préférence comporte une boucle de préhension.

14. Dispositif de maintien (1) selon l'une des revendications précédentes, **caractérisé en ce que** l'élément longiligne (18) comprend une zone centrale disposée entre des zones latérales et **en ce que** les zones latérales présentent un coefficient de friction moins élevé que la zone centrale.

15. Dispositif de maintien (1) selon l'une des revendications précédentes, **caractérisé en ce qu'**il comprend un dispositif de soutien intervertébral (19), notamment une cale intervertébrale (19) dont au moins la partie antérieure est apte à être disposée entre les lames sus et sous jacentes respectivement de deux vertèbres sus et sous jacentes.

16. Dispositif de maintien (1) selon la revendication 15, **caractérisé en ce que** le dispositif de soutien intervertébral (19) comporte au moins un moyen d'attache, de préférence disposé sur l'une de ses parois latérales, pour l'ancrage dudit élément longiligne.

## Patentansprüche

1. Haltevorrichtung (1), die dazu ausgelegt ist, einen bestimmten Zwischenwirbelabstand aufrechtzuerhalten, umfassend ein langgestrecktes, vorzugsweise im Wesentlichen flaches Element (18) von bestimmter Hauptbreite (1), mit einem ersten Ende (18a) und einem zweiten Ende (18b), welches geeignet ist, um Dornfortsätze herum angeordnet zu werden, wobei die Haltevorrichtung (1) **dadurch gekennzeichnet ist, dass** sie ferner umfasst:
a) einen starren Träger (2), der zwei zwischen einem vorderen Teil (5) und einem hinteren Teil (6) angebrachte Seitenteile (3, 4) umfasst, wobei das erste Ende (18a) mit dem hinteren Teil (6) fest verbunden ist oder geeignet ist, hiermit fest verbunden zu werden, und wobei das zweite Ende (18b) frei bleibt,
b) ein Blockierelement (7), das an den Seitenteilen (3, 4) verschieblich angebracht ist und einen vorderen (8) und einen hinteren (9) Durchgangsbereich mit dem vorderen (5) bzw. dem hinteren (6) Teil begrenzt, die den Durchgang des freien zweiten Endes (18b) in dem hinteren Durchgangsbereich (9), dann dem vorderen Durchgangsbereich (8) unter teilweisem Umschließen des Blockierelements (7) ermöglichen, so dass der Abschnitt (18c) des langgestreckten Elements (18), der sich im Wesentlichen zwischen seinem ersten Ende (18a) und dem Blockierelement (7) erstreckt, eine Schlaufe mit gegebenem Umfang (p) bildet und dass das Anlegen von entgegengesetzten Spannungen (T1, T2) an die Innenwände der Schlaufe das Bewegen des Blockierelements (7) zu dem vorderen Teil (5) und das Blockieren des Abschnitts (18c) des langgestreckten Elements (18) in dem vorderen Durchgangsbereich (8) zwischen dem Blockierelement (7) und dem vorderen Teil (5) des Trägers (2) bewirkt.

2. Haltevorrichtung (1) nach Anspruch 1, **dadurch gekennzeichnet, dass** der Innenabstand (d) zwischen den Seitenteilen (3, 4) im Wesentlichen in der Größenordnung von der Hauptbreite (l) des langgestreckten Elements (18) oder darunter liegt.

3. Haltevorrichtung (1) nach dem einen oder anderen der Ansprüche 1 und 2, **dadurch gekennzeichnet, dass** der vordere Teil (5) des Trägers (2) im Verhältnis zu dem Blockierelement (7) derart bemessen und angeordnet ist, dass er dem Blockierelement (7) als vorderer Anschlag dient, vorzugsweise der linke Rand (5a) und der rechte Rand (5b) des vorderen Teils (5), die auf beiden Seiten der Seitenteile (3, 4) vorspringen, den linken und rechten Rändern des Blockierelements (7) als Anschlag dienen.

4. Haltevorrichtung (1) nach einem der vorhergehenden Ansprüche, **dadurch gekennzeichnet, dass** das Blockierelement (7) eine mittlere Öffnung (10) aufweist, deren innere Seitenränder (10a, 10b) derart ausgespart sind, dass sie die Seitenteile (3, 4) des Trägers (2) aufnehmen und für das Gleiten des Blockierelements (7) auf den Seitenteilen (3, 4) zwischen dem vorderen Teil (5) und dem hinteren Teil (6) des Trägers als Führung dienen.

5. Haltevorrichtung (1) nach Anspruch 4, **dadurch gekennzeichnet, dass** der obere Umfang (12) der in Richtung des vorderen Teils (5) mündenden mittleren Öffnung (10) durch einen linken Rand (16a) und einen rechten Rand (16b) begrenzt ist und dass der linke Rand (16a) vom dem oberen Umfang (12) derart vorspringt, dass lediglich der linke Rand (16a) des Blockierelements (16) an dem vorderen Teil des Trägers in Anschlag gelangt, wobei zwischen dem rechten Rand (16b) und dem vorderen Teil (5) ein Spiel ausgebildet wird.

6. Haltevorrichtung (1) nach dem einem oder anderen der Ansprüche 4 und 5, **dadurch gekennzeichnet, dass** das Blockierelement vom hinteren Teil (6) zum vorderen Teil (5) ausgerichtete vertikale Nuten (17) umfasst, die an dem oberen Umfang (12) und eventuell dem unteren Umfang (13) der mittleren Öffnung (10) derart ausmünden, dass Klemmzähne (14) an dem oberen Umfang (12) und eventuell dem unteren Umfang (13) ausgebildet werden.

7. Haltevorrichtung (1) nach einem der Ansprüche 1 bis 4, **dadurch gekennzeichnet, dass** die in Richtung des vorderen Teils (5) des Trägers (2) mündende mittlere Öffnung (10) entlang wenigstens einem ihrer Ränder, dem linken (16a) oder rechten (16b), sägezahnförmige Zähne umfasst.

8. Haltevorrichtung (1) nach einem der vorhergehenden Ansprüche, **dadurch gekennzeichnet, dass** die inneren Eckbereiche des Trägers, die zwischen dem vorderen Teil und/oder dem hinteren Teil und dem einen oder anderen der beiden Seitenteile begrenzt sind, abgerundet sind.

9. Haltevorrichtung (1) nach einem der vorhergehenden Ansprüche, **dadurch gekennzeichnet, dass** der Träger aus einem Material besteht, das aus den folgenden Materialien, allein oder in Kombination, ausgewählt ist: rostfreier Stahl, Titan, Keramik, Polypropylen, Polyethylen, Polyethylen hoher Dichte, Polyetheretherketon PEEK, Chrom-Kobalt, PET.

10. Haltevorrichtung (1) nach einem der vorhergehenden Ansprüche, **dadurch gekennzeichnet, dass** das langgestreckte Element (18) durch das Informbringen von Multifilamentfäden und/oder von Monofilamenten durch Weben, Stricken oder Flechten, vorzugsweise durch Flechten von Multifilamentfäden erhalten wird.

11. Haltevorrichtung (1) nach einem der vorhergehenden Ansprüche, **dadurch gekennzeichnet, dass** das langgestreckte Element (18) aus einem Material besteht, das aus den folgenden Materialien, allein oder in Kombination, ausgewählt ist: Polypropylen, Polyethylenterephthalat, Polyethylen, Polyethylen hoher Dichte, Polyamid 6-6, 4-6 oder 12, Polyurethan, Polyetheretherketon (PEEK).

12. Haltevorrichtung (1) nach einem der vorhergehenden Ansprüche, **dadurch gekennzeichnet, dass** das langgestreckte Element (18) ein röhrenförmiges Geflecht ist, dessen erstes Ende (18a) und zweites Ende (18b) zugenäht wurden, um das röhrenförmige Geflecht in flache Form zu bringen.

13. Haltevorrichtung (1) nach einem der vorhergehenden Ansprüche, **dadurch gekennzeichnet, dass** das zweite Ende (18b) spitzenförmig ausgebildet ist, vorzugsweise eine Griffschlaufe umfasst.

14. Haltevorrichtung (1) nach einem der vorhergehenden Ansprüche, **dadurch gekennzeichnet, dass** das langgestreckte Element (18) einen zwischen Seitenbereichen angeordneten mittleren Bereich umfasst und dass die Seitenbereiche einen geringeren Reibungskoeffizienten als der mittlere Bereich aufweisen.

15. Haltevorrichtung (1) nach einem der vorhergehenden Ansprüche, **dadurch gekennzeichnet, dass** sie eine Zwischenwirbelstützvorrichtung (19), insbesondere einen Zwischenwirbelkeil (19) umfasst, dessen wenigstens vorderer Teil geeignet ist, zwischen den darüber und darunter liegenden Platten von zwei darüber bzw. darunter liegenden Wirbeln angeordnet zu werden.

16. Haltevorrichtung (1) nach Anspruch 15, **dadurch gekennzeichnet, dass** die Zwischenwirbelstützvorrichtung (19) wenigstens ein vorzugsweise an einer ihrer Seitenwände angeordnetes Befestigungsmittel zur Verankerung des langgestreckten Elements umfasst.

## Claims

1. A holding device (1) adapted for maintaining a predetermined intervertebral spacing comprising a preferably substantially flat, longilineal element (18), with a predetermined principal width (I), having a first (18a) and a second (18b) ends, capable of being positioned around spinous processes, said holding device (1) being **characterized in that** it also comprises:
a) a rigid support (2) including two lateral portions (3, 4) assembled between front (5) and rear (6) portions, said first end (18a) being joined or capable of being joined to said rear portion (6) and said second end (18b) remaining free,
b) a locking element (7) mounted so as to slide on said lateral portions (3, 4) and defining front (8) and rear (9) passage areas with the front (5) and rear (6) portions respectively, allowing passage of said second free end (18b) into the rear passage area (9) then into the front passage area (8) while partially surrounding said locking element (7) such that the portion (18c) of said longilineal element (18) extending substantially between its first end (18a) and the locking element (7) forms a loop with a given perimeter (p) and **in that** the application of opposite tensions (T1, T2) on the inner walls of the loops causes the displacement of said locking element (7) toward said front portion (5) and the locking of the portion (18c) of the longilineal element (18) in the front passage area (8) between said locking element (7) and the front portion (5) of the support (2).

2. The holding device (1) according to Claim 1, **characterized in that** the inner distance (d) separating the lateral portions (3, 4) is substantially of the order of the principal width (I) of said longilineal element (18), or less.

3. The holding device (1) according to one or the other of Claims 1 and 2, **characterized in that** the front portion (5) of the support (2) is sized and positions with respect to the locking element (7) so as to act as a forward stop for said locking element (7), the left (5a) and right (5b) edges of the front portion (5) preferably projecting to either side of the lateral portions (3, 4) act as a stop on the left and right edges of the locking element (7).

4. The holding device (1) according to one of the foregoing claims, **characterized in that** the locking element (7) includes a central opening (10) the inner lateral edges whereof (10a, 10b) are arranged so as to accommodate the lateral portions (3, 4) of the support (2) and to serve as a guide for the sliding of the locking element (7) on the lateral portions (3, 4) between the front (5) and rear (6) portions of the support (2).

5. The holding device (1) according to Claim 4, **characterized in that** the upper periphery (12) of the central opening (10) leading toward the front portion (5) is defined by left (16a) and right (16b) edges and **in that** the left edge (16a) projects from said upper periphery (12) so that only the left edge (16a) of the locking element (16) comes to bear against the front portion (5) of the support (2) providing play between the right edge (16b) and the front portion (5).

6. The holding device (1) according to one or the other of Claims 4 and 5, **characterized in that** the locking element includes vertical grooves (17), oriented from the rear portion (6) toward the front portion (5), reaching to the upper periphery (12) and possibly the lower periphery (13) of the central opening (10) so as to provide clamping teeth (14) on the upper periphery (12) and possibly on the lower periphery (13).

7. The holding device (1) according to any one of Claims 1 through 4, **characterized in that** the central opening (10) opening toward the front portion (5) of the support (2) comprises teeth shaped like saw teeth along at least one of its left (16a) or right (16b) edges.

8. The holding device (1) according to one of the foregoing claims, **characterized in that** the inner angular areas of the support defined between the front and/or rear portions and one or the other of the two lateral portions are rounded.

9. The holding device (1) according to one of the foregoing claims, **characterized in that** the support is made of a material selected, alone or in combination among the following materials: stainless steel, titanium, ceramic, polypropylene, polyethylene, high density polyethylene, polyether etherketone (PEEK), chrome-cobalt alloy, PET.

10. The holding device (1) according to one of the foregoing claims, **characterized in that** the longilineal element (18) is obtained by forming monofilament and/or multifilament yarns, by weaving, knitting or braiding, preferably by braiding of multifilament yarns.

11. The holding device (1) according to one of the foregoing claims, **characterized in that** the longilineal element (18) is made of a material selected along or in combination among the following materials: polypropylene, polyethylene terephtalate, polyethylene, high density polyethylene, polyamide 6-6, 4-6 or 12, polyurethane, polyether etherketone (PEEK).

12. The holding device (1) according to one of the foregoing claims, **characterized in that** the longilineal element (18) is a tubular braid the first (18a) and second (18b) ends whereof are sewn so as to flatten the tubular braid.

13. The holding device (1) according to one of the foregoing claims, **characterized in that** the second end (18b) is made in the form of a point and preferably comprises a gripping loop.

14. The holding device (1) according to one of the foregoing claims, **characterized in that** the longilineal element (18) includes a central area positioned between the lateral areas and **in that** the lateral areas exhibit a lower coefficient of friction than the central area.

15. The holding device (1) according to one of the foregoing claims, **characterized in that** it includes an intervertebral support device (19), particularly an intervertebral wedge (19) at least the anterior part whereof is capable of being positioned between the over- and underlying laminae respectively of two over- and underlying vertebrae.

16. The holding device (1) according to Claim 15, **characterized in that** the intervertebral support device (19) comprises at least one attachment means, preferably positioned on one of its lateral walls, for anchoring said longilineal element.
